(19) Europäisches Patentamt
European Patent Office
Office européen des brevets

(11) Veröffentlichungsnummer: **0 007 582**
**B1**

(12) # EUROPÄISCHE PATENTSCHRIFT

(45) Veröffentlichungstag der Patentschrift: **25.11.81**

(51) Int. Cl.³: **A 24 B 15/34**, C 07 C 49/743

(21) Anmeldenummer: **79102546.3**

(22) Anmeldetag: **19.07.79**

(54) **Kompositionen und Verfahren zur Verstärkung, Modifizierung oder Verbesserung der organoleptischen Eigenschaften von Tabakerzeugnissen und dazu verwendbare Verbindungen.**

(30) Priorität: **25.07.78 US 927800**

(43) Veröffentlichungstag der Anmeldung: **06.02.80 Patentblatt 80/3**

(45) Bekanntmachung des Hinweises auf die Patenterteilung: **25.11.81 Patentblatt 81/47**

(84) Benannte Vertragsstaaten: **CH DE FR GB NL**

(56) Entgegenhaltungen:
**DE-A-2 724 180**
**FR-A-2 128 744**
**US-A-4 029 108**
**L. BARDOU et al.: »Recherches dans la série des azoles. XVI. Pyrazoles bicycliques« Bulletin de la société chimique de France, 1967, Nr. 1, Seiten 289–294 Paris, FR.**

(73) Patentinhaber: **L. GIVAUDAN & CIE Société Anonyme, CH-1214 Vernier-Genève (CH)**

(72) Erfinder: **Flynn, Cormack, 291 Grove Street, Ramsey, N.J. 07446 (US)**
Erfinder: **Fredericks, Glen R., 15 Barkley Avenue, Clifton, N.J. 07011 (US)**
Erfinder: **Hochstetler, Alan R., 109 East First Street, Clifton, N.J. 07011 (US)**

(74) Vertreter: **Urech, Peter, Dr. et al, Grenzacherstrasse 124 Postfach 601, CH-4002 Basel (CH)**

**EP 0 007 582 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Kompositionen und Verfahren zur Verstärkung, Modifizierung oder Verbesserung der organoleptischen Eigenschaften von Tabakerzeugnissen und dazu verwendbare Verbindungen

Es wurde gefunden, daß die organoleptischen Eigenschaften von Tabakerzeugnissen verbessert werden können durch Zusatz eines 2-Hydroxymethylencyclohexanons der allgemeinen Formel

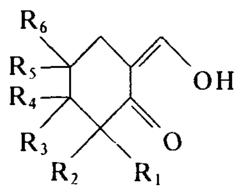

(I)

worin $R_1$ Wasserstoff, Methyl, Äthyl, Propyl (alle Propyl-Reste), Allyl oder Butyl (alle Butyl-Reste) bedeutet; $R_2$, $R_3$, $R_4$, $R_5$ und $R_6$ je Wasserstoff, Methyl oder Äthyl darstellen und die Gesamtzahl der Kohlenstoffatome $R_1+R_2+R_3+R_4+R_5+R_6$ fünf nicht übersteigt.

»Tabakerzeugnis« ist eine in der Branche übliche, allgemeine Bezeichnung, die aber nicht nur Tabak selbst umfaßt, sondern auch Tabaknebenprodukte, wie rekonstituierte und homogenisierte Blätter und Stengel, Tabaksurrogate (z. B. Salat- und Kohlblätter, etc.), Materialien, die bei der Tabakverarbeitung gebraucht werden, wie Papier, Filter etc. und für Tabakerzeugnisse verwendete Geschmackskompositionen. Unter die Bezeichnung »Tabakerzeugnis« fallen Zigarettentabak, Zigarrentabak, Kautabak und Pfeifentabak, etc.

Der Zusatz eines 2-Hydroxymethylencyclohexanons der Formel I zu einer Tabakmischung verbessert sowohl den Geruch des frischen Tabaks als auch den Geruch und den Geschmack des Tabaks beim Rauchen. Ein Vergleich von behandeltem mit unbehandeltem Tabak zeigt, daß der Geruch der Mischungen, die 2-Hydroxymethylencyclohexanons der Formel I enthalten, gegenüber den unbehandelten verstärkt, abgerundeter und leichter ist.

Die Unterschiede zwischen den behandelten und unbehandelten Tabaken werden noch auffälliger beim Rauchen. Die unbehandelten Zigaretten weisen beim Rauchen eine unerwünschte Herbheit auf, ein Effekt, der durch den Zusatz eines 2-Hydroxymethylencyclohexanons der Formel I verringert wird. Die behandelten Zigaretten ergeben beim Rauchen einen weicheren, leichteren und abgerundeteren Geschmack, weshalb sie den unbehandelten eindeutig vorgezogen werden.

Die Hydroxymethylen-Verbindungen der Formel I können in an sich bekannter Weise, wie in Organic Synthesis, Coll. Vol. 4, John Wiley and Sons, Inc. New York (1963), 536 beschrieben, aus dem entsprechenden Keton durch basenkatalysierte Reaktion mit einem Formiat, z. B. einem Alkylformiat wie Äthylformiat, hergestellt werden, z. B. gemäß:

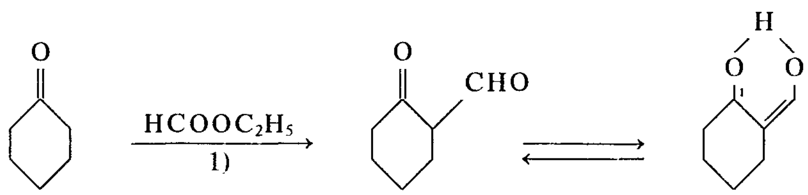


(II)

1) Natriumhydrid oder Natriumäthoxid, wie gemäß Organic Synthesis loc. cit oder andere Alkalimetallalkoxide (z. B. Natriummethoxid, Kalium-t-butoxid etc.) und Basen von Alkaliamid-Typ (z. B. Natriumamid, Lithiumdiisopropylamid und ähnliche).

Die Reaktion kann in irgendeinem aprotischen Lösungsmittel, wie z. B. Toluol, Pentan, Heptan, Hexan, Cyclohexan, Benzol, THF, Äther etc. und bei Temperaturen zwischen 0 und 50°C, z. B. bei Raumtemperatur, aber auch bei höheren Temperaturen durchgeführt werden.

Die Formylgruppe scheint ausschließlich als das Hydroxymethylen-Tautomere vorzuliegen, da zwischen dem Hydroxyl-Wasserstoffatom und dem Carbonyl-Sauerstoffatom eine Wasserstoff-Bindung besteht. Im Gegensatz dazu ist diese tautomere Form beispielsweise im Falle des 2,6,6-Trimethyl-3-Oxo-cyclohex-1-en-carbaldehyds der DE-A-2 724 180 nicht möglich, so daß dort klarerweise eben ein Aldehyd vorliegt.

Durch Zusatz eines 2-Hydroxymethylencyclohexanon-Derivates der Formel I zu einer Tabakmischung wird die Rauchcharakteristik dieser Mischung deutlich verbessert. Die Herbheit des Geschmacks, die man beim Rauchen der unbehandelten Mischung empfindet, wird stark vermindert.

Von den Verbindungen der Formel I sind jene bevorzugt, worin $R_2$, $R_3$, $R_4$, $R_5$ und $R_6$ entweder Wasserstoff oder Methyl bedeuten, und die Gesamtzahl der Kohlenstoffatome in den Substituenten ($R_1$ bis $R_6$) vier nicht übersteigt. Während eine Anzahl Verbindungen dieser Gruppe (z. B. 6-Hydroxymethylen-2-methyl-2-propylcyclohexanon, 2-Allyl-6-hydroxymethylen-2-methylcyclohexa-

non, 6-sec. Butyl-2-hydroxy-methylencyclohexanon) bei Zusatz zu einer Tabakmischung die verbesserten Raucheigenschaften ergeben, zeigen Verbindungen ähnlicher Struktur, die aber nicht zu dieser Gruppe gehören, wie 4-t-Butyl-2-hydroxymethylencyclohexanon oder 2-Hydroxymethylen-4-isopropylcyclohexanon, diese erwünschten Eigenschaften nicht.

Besonders bevorzugt sind jene Verbindungen der Formel I, worin die Substituenten $R_1$ bis $R_6$ Wasserstoff oder Methyl bedeuten und die Gesamtzahl der Methylgruppen nicht größer als drei ist. Diese Verbindungen (z. B. 2-Hydroxymethylencyclohexanon, 2-Hydroxymethylen-6-methylcyclohexanon, 2,3-Dimethyl-6-hydroxymethylencyclohexanon, 2-Hydroxymethylen-4,4,6-trimethylcyclohexanon etc.) sind besonders wirksam, um die Herbheit zu vermindern und einen milderen, leichteren und abgerundeteren Geschmack beim Rauchen zu erzielen.

Überraschenderweise verbessern eng verwandte Verbindungen, wie 3,6-Dimethyl-2-hydroxymethylencyclohexanon und 2-Hydroxymethylen-3,5,5-trimethylcyclohexanon das Tabakaroma nicht gleichermaßen wie die Verbindungen der Formel I.

Die Menge des 2-Hydroxymethylencyclohexanon-Derivates der Formel I, die zweckmäßigerweise zugesetzt wird, kann von verschiedenen Faktoren, einschließlich der gewünschten Wirkung, der Art und der Menge anderer, gleichzeitig verwendeter Zusätze und/oder der persönlichen Vorliebe des Aromatikers, abhängen. Schon Mengen von 0,01 ppm, bezogen auf das Gewicht des Tabaks, erweisen sich als wirksam, während aber auch Mengen von 1000 ppm noch verwendbar sind. Bevorzugt werden jedoch Mengen von 0,1 ppm bis 100 ppm eingesetzt; Mengen zwischen 1 ppm und 10 ppm sind besonders bevorzugt.

Die oben vorgeschlagenen Grenzen sollen selbstverständlich nur die bevorzugten Mengen andeuten; diese sind jedoch auch von der Geschicklichkeit des Aromatikers und der Wirkung, die er erzielen will, abhängig.

Die Verbindungen I können dem Tabakprodukt (Zigarettenpapier, etc.) nach den, dem Fachmann bekannten Methoden, z. B. Zerstäuben, Eintauchen, Überziehen, etc. zugesetzt oder beigemischt werden.

Die folgenden Beispiele sollen als Veranschaulichung der bevorzugten Ausführungsformen der vorliegenden Erfindung und nicht als Einschränkung verstanden werden. Sie sollen also auch daraus ableitbaren Variationen umfassen, die für den Fachmann naheliegend sind.

## Beispiel 1

Das Beispiel beschreibt eine Methode zur Herstellung der 2-Hydroxymethylencyclohexanon-Derivate.

### A. 2-Hydroxymethylencyclohexanon

Ein Gemisch von 105 g Natriumhydrid (50%ige Öl-Dispersion, 2,1 Mol), 200 g Äthylformiat (2,7 Mol), 5 ml trockenem Äthanol und 3,5 l Hexan wird bei 20°C unter Stickstoff gerührt und während 2¾ Stunden 200 g Cyclohexanon (2 Mol) zugetropft. Bei Raumtemperatur wird 20 Stunden weitergerührt und gleichzeitig genügend Wasser zugegeben, um das Reaktionsgemisch zu lösen. Die Phasen werden getrennt, und die organische Phase wird zweimal mit 5%iger wäßriger Natriumhydroxid-Lösung gewaschen. Die wäßrige Phasen werden vereinigt, mit 25%iger wäßriger Schwefelsäure sauer gestellt und dreimal mit Hexan extrahiert. Anschließend wird die organische Phase zweimal mit Wasser gewaschen und mit einer 4″-Vigreux-Kolonne (1″=2,54 cm) bei einem Rücklaufverhältnis von 1 : 1 destilliert. Dabei werden 318 g (63% Ausbeute) 2-Hydroxymethylencyclohexanon erhalten; Sdp. 47–48°C/0,5 mmHg. [In Organic Synthesis, Coll. Vol. 4, John Wiley and Sons, Inc., New York 1963, S. 536 ff. ist ein ähnliches Verfahren beschrieben.]

Auf ähnliche Weise wurden aus den entsprechenden Ketonen die in Tabelle I des folgenden Beispiels 2 aufgeführten 2-Hydroxymethylencyclohexanon-Derivate hergestellt.

## Beispiel 2

Dieses Beispiel beschreibt die Vorzüge der Zigaretten, die durch Zusatz der 2-Hydroxymethylencyclohexanone resultieren.

Zur Herstellung der Zigaretten wurde folgende Tabakmischung verwendet:

| Verwendeter Tabak | Gewichtsanteil |
|---|---|
| Bright | 60 |
| Burley | 20 |
| Oriental | 10 |
| Bright Stems | 10 |
| | 100 |

Die Hälfte der Zigaretten wurde zur Kontrolle gebraucht und wiesen keine Zusätze auf. Die andere Hälfte der Zigaretten wurde mit 2-Hydroxymethylencyclohexanonen behandelt. Die Menge des zusatzes betrug etwa 10 ppm.

Die Zigaretten wurden von einem Panel verglichen, das nicht wußte, welche Zigaretten behandelt waren. Die Zigaretten, die 2-Hydroxymethylencyclohexanone enthielten, wurden als weicher, tabakähnlicher und als von allgemein besserer Qualität als die Kontroll-Zigaretten beschrieben.

In Tabelle I sind mehrere untersuchte Verbindungen aufgeführt, zusammen mit den Ausbeuten, die bei der Herstellung nach einem dem Beispiel 1 analogen Verfahren erzielt wurden, ihren Siedepunkten bei 0,5 mmHg und der Wirkung beim Rauchen, wenn sie, wie oben beschrieben, geprüft werden. Die mit Stern versehenen Verbindungen sind Vergleichsverbindungen (fallen also nicht unter die Formel I).

Tabelle I

| Verbindungen | Wirkung beim Rauchen |
|---|---|
| 1. 2-Hydroxymethylencyclohexanon (63%; 47–48°C) | leicht, mild, gutes Mundgefühl; vermindert Herbheit, verstärktes Tabakaroma. |
| 2. 2-Hydroxymethylen-6-methylcyclohexanon (70%; 52–53°C) | vermindert Herbheit, leicht, verstärkt Eindruck des Tabakaromas. |
| 3. 2-Hydroxymethylen-5-methylcyclohexanon (70%; 58–60°C) | leicht, vermindert Herbheit, mild |
| 4. 2-Hydroxymethylen-4-methylcyclohexanon (84%; 61–62°C) | vermindert Herbheit und Bitterkeit, mild, leicht. |
| 5. 2,3-Dimethyl-6-hydroxymethylencyclohexanon (90%; 53–55°C) | leicht, mild, vermindert Herbheit, verbessertes Tabakaroma. |
| 6. * 3,6-Dimethyl-2-hydroxymethylencyclohexanon[1]) (74%; 63–64°C) | neutralisiert Rauch vollständig. |
| 7. 2-Hydroxymethylen-4,4,6-trimethylcyclohexanon (88%; 56–57°C) | milderer, vollerer und reicherer Geschmack, vermindert Herbheit. |
| 8. * 2-Hydroxymethylen-3,5,5-trimethylcyclohexanon[2]) (85%; 56–57°C) | schlechter Geschmack, abgestanden. |
| 9. * 2-Hydroxymethylen-4-isopropylcyclohexanon (87%; 72–73°C) | schlechter Geschmack, herb. |
| 10. * 4-t-Butyl-2-hydroxymethylencyclohexanon[3]) (89%; 84–85°C) | keine Steigerung des Tabakaromas, nicht leicht. |
| 11. 6-sec.-Butyl-2-hydroxymethylencyclohexanon[3]) (74%; 88–89°C) | ausgeglichen, leicht, vermindert Herbheit. |
| 12. 6-Hydroxymethylen-2-methyl-2-propylcyclohexanon (71%; 80–81°C) | mild, leicht, voll, vermindert Herbheit. |
| 13. 2-Allyl-6-hydroxymethylen-2-methylcyclohexanon (77%; 68–69°C) | leichte Nebennote, Herbheit. vermindert |
| 14. 2-Äthyl-6-hydroxymethylen-2-methylcyclohexanon (71%; 60–61°C) | weniger-herb, leicht. |

*) Vergleichsverbindung.

[1]) Siehe z. B. A. Melera, D. Arigoni, A. Eschenmoser, O. Jeger, L. Ruzicka, Helv. Chim. Acta 39, 441–8 (1956); ohne Hinweis auf irgendwelche organoleptischen Eigenschaften der Verbindung.

[2]) Y. Bessiere, F. Ouar, J. Labelled Compds. 11 (1), 3–9 (1975); ohne Hinweis auf irgendwelche organoleptischen Eigenschaften der Verbindung.

[3]) V. M. Potapov, V. G. Bakjmut-skaya, I. G. Il'ina, E. G. Rukhadze, G. I. Vinnik, Zh. Obshch. Khim 46 (9), 2117–21 (1976); ohne Hinweis auf irgendwelche organoleptischen Eigenschaften der Verbindung.

## Beispiel 3

Dieses Beispiel beschreibt die Vorzüge der Tabakgeschmackstoffkompositionen, die 2-Hydroxymethylencyclohexanon-Verbindungen der Formel I enthalten.

Es wurden folgende Gemische hergestellt:

| Bestandteile | Gewichtsteile A | B | C |
|---|---|---|---|
| »Deertongue«-Blätter-Extrakt (Liatris) | 10 | 10 | 10 |
| Wasser | 20 | 20 | 20 |
| Alkohol | 47 | 37 | 37 |
| Cumarin | 5 | 5 | 5 |
| Äthyloxyhydrat (Rumäther) | 15 | 15 | 15 |
| Linalool | 1 | 1 | 1 |
| δ-Decalacton | 1 | 1 | 1 |
| α-Ionon | 1 | 1 | 1 |
| 2-Hydroxymethylencyclohexanon | – | 10 | – |
| 6-Methyl-2-hydroxymethylencyclohexanon | – | – | 10 |
| | 100 | 100 | 100 |

Durch Behandlung mit einer der obigen Formulierungen resultieren drei Gruppen von Zigaretten. Die verwendete Menge an I betrug 10 ppm. [Die geeigneten Konzentrationen der Verbindungen I in Tabakgeschmacksmischungen können zwischen 0,1 und 100% liegen.]

Die Zigaretten wurden durch ein Test-panel untersucht. Jene Zigaretten, die mit dem Gemisch A behandelt waren, das keine 2-Hydroxymethylencyclohexanone enthielt, wurden als herb und stark beurteilt.

Im Vergleich zu A wurden die mit Gemisch B behandelten Zigaretten als milder beurteilt. Die Rauchcharakteristik wurde, verglichen mit A, als verbessert bezeichnet. In ähnlicher Weise wurden die mit Gemisch C behandelten Zigaretten als milder, abgerundeter und schmackhafter beschrieben als jene, die Mischung A enthielten.

Ähnliche Ergebnisse ergeben sich, wenn die spezifischen 2-Hydroxymethylencyclohexanone in den oben genannten Formulierungen durch andere Verbindungen der Formel I ersetzt werden.

**Patentansprüche**

1. Komposition zur Verstärkung, Modifizierung oder Verbesserung der organoleptischen Eigenschaften von Tabakerzeugnissen, gekennzeichnet durch einen Gehalt an einem 2-Hydroxymethylencyclohexanon der allgemeinen Formel

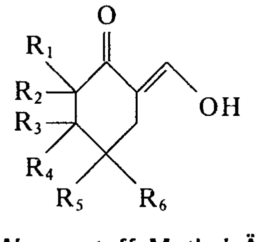

(I)

worin $R_1$ Wasserstoff, Methyl, Äthyl, Propyl, Allyl oder Butyl bedeutet, $R_2$, $R_3$, $R_4$, $R_5$ und $R_6$ Wasserstoff, Methyl oder Äthyl darstellen und die Gesamtzahl der Kohlenstoffatome $R_1+R_2+R_3+R_4+R_5+R_6$ fünf nicht übersteigt,
und einen oder mehrere weitere Bestandteile von Riech- und/oder Geschmackstoffkompositionen.

2. Komposition gemäß Anspruch 1, worin $R_2$, $R_3$, $R_4$, $R_5$ und $R_6$ Wasserstoff oder Methyl darstellen und die Gesamtzahl der Kohlenstoffatome $R_1+R_2+R_3+R_4+R_5+R_6$ vier nicht übersteigt.

3. Komposition gemäß Anspruch 2, gekennzeichnet durch einen Gehalt an 2-Hydroxymethylen-6-methyl-6-propylcyclohexanon.

4. Komposition gemäß Anspruch 2, worin $R_1$, $R_2$, $R_3$, $R_4$, $R_5$ und $R_6$ Wasserstoff oder Methyl darstellen und die Gesamtzahl der Methylreste drei nicht übersteigt.

5. Komposition gemäß Anspruch 4, gekennzeichnet durch einen Gehalt an 2-Hydroxymethylencyclohexanon.

6. Komposition gemäß Anspruch 4, gekennzeichnet durch einen Gehalt an 2-Hydroxymethylen-6-methylcyclohexanon.
7. Komposition gemäß Anspruch 4, gekennzeichnet durch einen Gehalt an 2-Hydroxymethylen-5-methylcyclohexanon.
8. Komposition gemäß Anspruch 4, gekennzeichnet durch einen Gehalt an 2-Hydroxymethylen-4-methylcyclohexanon.
9. Komposition gemäß Anspruch 4, gekennzeichnet durch einen Gehalt an 2,3-Dimethyl-6-hydroxymethylencyclohexanon.
10. Komposition gemäß Anspruch 4, gekennzeichnet durch einen Gehalt an 2-Hydroxymethylen-4,4,6-trimethylcyclohexanon.
11. Verwendung einer Verbindung der allgemeinen Formel

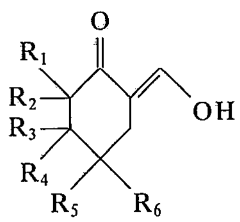

(I)

worin $R_1$ Wasserstoff, Methyl, Äthyl, Propyl, Allyl oder Butyl bedeutet; $R_2$, $R_3$, $R_4$, $R_5$ und $R_6$ Wasserstoff, Methyl oder Äthyl darstellen und die Gesamtzahl der Kohlenstoffatome $R_1+R_2+R_3+R_4+R_5+R_6$ fünf nicht übersteigt,
zur Verstärkung, Modifizierung oder Verbesserung der organoleptischen Eigenschaften von Tabakerzeugnissen.
12. Eine Verbindung, ausgewählt aus 2-Hydroxymethylen-4,4,6-trimethylcyclohexanon, 6-Hydroxymethylen-2-methyl-2-propylcyclohexanon, 2-Allyl-6-hydroxymethylen-2-methylcyclohexanon und 2-Äthyl-6-hydroxymethylen-2-methylcyclohexanon.
13. 2-Hydroxymethylen-4,4,6-trimethylcyclohexanon, 6-Hydroxymethylen-2-methyl-2-propylcyclohexanon, 2-Allyl-6-hydroxymethylen-2-methylcyclohexanon oder 2-Äthyl-6-hydroxymethylen-2-methylcyclohexanon als Zusatz für Tabakerzeugnisse.

## Claims

1. Composition for intensifying, modifying or improving the organoleptic properties of tobacco products, characterised by a content of a 2-hydroxymethylenecyclohexanone of the general formula

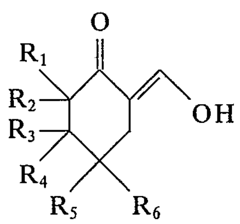

(I)

wherein $R_1$ signifies hydrogen, methyl, ethyl, propyl, allyl or butyl, $R_2$, $R_3$, $R_4$, $R_5$ and $R_6$ represent hydrogen, methyl or ethyl and the total number of carbon atoms $R_1+R_2+R_3+R_4+R_5+R_6$ does not exceed five,
and one or more additional ingredients of odorant and/or flavouring substance compositions.
2. Composition according to claim 1, wherein $R_2$, $R_3$, $R_4$, $R_5$ and $R_6$ represent hydrogen or methyl and the total number of carbon atoms $R_1+R_2+R_3+R_4+R_5+R_6$ does not exceed four.
3. Composition according to claim 2, characterised by a content of 2-hydroxymethylene-6-methyl-6-propylcyclohexanone.
4. Composition according to claim 2, wherein $R_1$, $R_2$, $R_3$, $R_4$, $R_5$ and $R_6$ represent hydrogen or methyl and the total number of methyl residues does not exceed three.
5. Composition according to claim 4, characterised by a content of 2-hydroxymethylenecyclohexanone.
6. Composition according to claim 4, characterised by a content of 2-hydroxymethylene-6-methylcyclohexanone.
7. Composition according to claim 4, characterised by a content of 2-hydroxymethylene-5-methylcyclohexanone.
8. Composition according to claim 4, characterised by a content of 2-hydroxymethylene-4-methylcyclohexanone.

9. Composition according to claim 4, characterised by a content of 2,3-dimethyl-6-hydroxymethylenecyclohexanone.

10. Composition according to claim 4, characterised by a content of 2-hydroxymethylene-4,4,6-trimethylcyclohexanone.

11. Use of a compound of the general formula

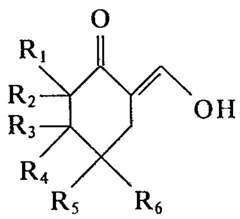

(I)

wherein $R_1$ signifies hydrogen, methyl, ethyl, propyl, allyl or butyl; $R_2$, $R_3$, $R_4$, $R_5$ and $R_6$ represent hydrogen, methyl or ethyl and the total number of carbon atoms $R_1+R_2+R_3+R_4+R_5+R_6$ does not exceed five,
for intensifying, modifying or improving the organoleptic properties of tobacco products.

12. A compound selected from 2-hydroxymethylene-4,4,6-trimethylcyclohexanone, 6-hydroxymethylene-2-methyl-2-propylcyclohexanone, 2-allyl-6-hydroxymethylene-2-methylcyclohexanone and 2-ethyl-6-hydroxymethylene-2-methylcyclohexanone.

13. 2-Hydroxymethylene-4,4,6-trimethylcyclohexanone, 6-hydroxymethylene-2-methyl-2-propylcyclohexanone, 2-allyl-6-hydroxymethylene-2-methylcyclohexanone or 2-ethyl-6-hydroxymethylene-2-methylcyclohexanone as an additive for tobacco products.

**Revendications**

1. Composition pour renforcer, modifier ou améliorer les propriétés organoleptiques des produits de tabac, caractérisée en ce qu'elle contient une 2-hydroxyméthylène-cyclohexanone de formule générale

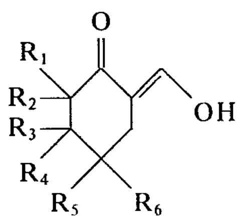

(I)

dans laquelle $R_1$ représente l'hydrogène, un groupe méthyle, éthyle, propyle, allyle ou butyle, $R_2$, $R_3$, $R_4$, $R_5$ et $R_6$ représentent l'hydrogène, des groupes méthyle ou éthyle, et le nombre des atomes de carbone de $R_1+R_2+R_3+R_4+R_5+R_6$ ne dépasse pas 5,
et un ou plusieurs autres constituants de compositions odoriférantes et/ou aromatisantes.

2. Composition selon la revendication 1, caractérisée en ce que $R_2$, $R_3$, $R_4$, $R_5$ et $R_6$ représentent l'hydrogène ou des groupes méthyles et le nombre total des atomes de carbone de $R_1+R_2+R_3+R_4+R_5+R_6$ ne dépasse pas 4.

3. Composition selon la revendication 2, caractérisée en ce qu'elle contient de la 2-hydroxyméthylène-6-méthyl-6-propylcyclohexanone.

4. Composition selon la revendication 2, caractérisée en ce que $R_1$, $R_2$, $R_3$, $R_4$, $R_5$ et $R_6$ représentent l'hydrogène ou des groupes méthyles et le nombre total des groupes méthyles ne dépasse pas 3.

5. Composition selon la revendication 4, caractérisée en ce qu'elle contient de la 2-hydroxyméthylène-cyclohexanone.

6. Composition selon la revendication 4, caractérisée en ce qu'elle contient de la 2-hydroxyméthylène-6-méthyl-cyclohexanone.

7. Composition selon la revendication 4, caractérisée en ce qu'elle contient de la 2-hydroxyméthylène-5-méthyl-cyclohexanone.

8. Composition selon la revendication 4, caractérisée en ce qu'elle contient de la 2-hydroxyméthylène-4-méthyl-cyclohexanone.

9. Composition selon la revendication 4, caractérisée en ce qu'elle contient de la 2,3-diméthyl-6-hydroxyméthylène-cyclohexanone.

10. Composition selon la revendication 4, caractérisée en ce qu'elle contient de la 2-hydroxyméthylène-4,4,6-triméthyl-cyclohexanone.

11. Utilisation d'un composé de formule générale

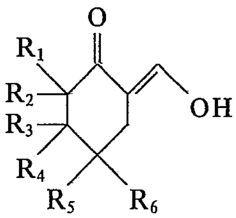

(I)

dans laquelle $R_1$ représente l'hydrogène, un groupe méthyle, éthyle, propyle, allyle ou butyle; $R_2$, $R_3$, $R_4$, $R_5$ et $R_6$ représentent l'hydrogène, des groupes méthyles ou éthyles et le nombre total des atomes de carbone de $R_1+R_2+R_3+R_4+R_5+R_6$ ne dépasse pas 5,
pour renforcer, modifier ou améliorer les propriétés organoleptiques de produits de tabac.

12. Un composé choisi parmi la 2-hydroxyméthylène-4,4,6-triméthylcyclohexanone, la 6-hydroxyméthylène-2-méthyl-2-propyl-cyclohexanone, la 2-allyl-6-hydroxyméthylène-2-méthylcyclohexanone et la 2-éthyl-6-hydroxyméthylène-2-méthylcyclohexanone.

13. La 2-hydroxyméthylène-4,4,6-triméthylcyclohexanone, la 6-hydroxyméthylène-2-méthyl-2-propylcyclohexanone, la 2-allyl-6-hydroxyméthylène-2-méthylcyclohexanone ou la 2-éthyl-6-hydroxyméthylène-2-méthylcyclohexanone en tant qu'additifs à des produits de tabac.